Europäisches Patentamt

European Patent Office

Office européen des brevets

(19)

(11) Numéro de publication : **0 679 891 A2**

# (12) DEMANDE DE BREVET EUROPEEN

(21) Numéro de dépôt : **95110811.7**

(22) Date de dépôt : **16.11.90**

(51) Int. Cl.⁶ : **G01N 33/543,** G01N 35/02, B03C 1/00

Cette demande a été déposée le 11 - 07 - 1995 comme demande divisionnaire de la demande mentionnée sous le code INID 60.

(30) Priorité : **17.11.89 FR 8915095**

(43) Date de publication de la demande :
**02.11.95 Bulletin 95/44**

(60) Numéro de publication de la demande initiale en application de l'article 76 CBE : **0 454 826**

(84) Etats contractants désignés :
**AT BE CH DE DK ES FR GB GR IT LI LU NL SE**

(71) Demandeur : **Laboratoires Merck-Clévenot**
**5-9 Rue Anquetil**
**F-92736 Nogent-sur-Marne (FR)**

(72) Inventeur : **Uzan, Michel**
**113 avenue Franklin Roosevelt**
**F-93320 Les Pavillons sous Bois (FR)**
Inventeur : **Gicquel, Thierry**
**31 rue Molière**
**F-95000 Jouy le Moutier (FR)**
Inventeur : **Lentwojt, Edouard**
**5 allée Marcel Philippe**
**F-60340 Saint Leu d'Esserent (FR)**
Inventeur : **Narminio, Dario**
**10 rue du 11 novembre 1918**
**F-95380 Louvres (FR)**

(74) Mandataire : **Orès, Bernard et al**
**Cabinet ORES6, Avenue de Messine**
**F-75008 Paris (FR)**

(54) **Réactif de diagnostic immunologique.**

(57) Réactifs de diagnostic immunologique, aptes à être utilisés dans un appareil d'exécution automatique d'un immunodosage en plusieurs étapes successives, d'au moins une substance biologique dans une pluralité d'échantillons biologiques.

Lesdits réactifs de diagnostic immunologique sont constitués de billes magnétiques recouvertes d'une substance se liant spécifiquement à la substance à détecter, en suspension dans un liquide approprié, constituées d'une matrice organique renfermant une charge magnétique, lesdites billes ayant un rapport masse de matière magnétisable/masse de matière non magnétisable compris entre 60 et 70 %.

EP 0 679 891 A2

La présente invention est relative à des réactifs de diagnostic immunologique, aptes à être utilisés dans un appareil d'exécution automatique d'un immunodosage en plusieurs étapes successives, d'au moins une substance biologique dans une pluralité d'échantillons biologiques.

L'invention s'applique plus particulièrement, mais non limitativement, à la détection simultanée sur un même échantillon de ligands, d'antiligands, d'haptènes ou de toute autre substance biologique éventuellement présente dans le fluide biologique à analyser.

Les méthodes de détermination de la présence ou de la concentration de ligands ou de substances biologiques dans des fluides biologiques, par voie immunologique, sont à présent bien connues ; elles sont basées sur la formation d'un complexe entre la substance à déterminer et un ou des anticorps, l'un des composants du complexe pouvant être marqué notamment par une enzyme, pour permettre sa détection et/ou son analyse quantitative après séparation de l'antigène ou de l'anticorps marqué complexé, de l'antigène ou de l'anticorps marqué non complexé.

Le principe des méthodes RIA et ELISA est également bien connu.

La technique ELISA (Enzyme linked immunosorbent assay), notamment, est une méthode immuno-enzymatique, d'une très grande sensibilité, de titrage d'anticorps, par exemple, qui consiste à utiliser un complexe immunoadsorbant constitué notamment par un antigène fixé sur un support solide, pour capter les anticorps spécifiques contenus dans le milieu biologique, tel que sérum, à tester, l'immun-complexe ainsi obtenu étant détecté par un anticorps anti-espèce marqué par une enzyme, après quoi on ajoute un substrat spécifique de l'enzyme, dont la dégradation par l'enzyme fait apparaître un produit coloré ; l'intensité de la coloration est proportionnelle à la quantité d'enzyme qui réagit et donc à la quantité d'anticorps présente dans le milieu biologique testé ; l'intensité de la coloration peut être appréciée à l'oeil nu ou mesurée par tout moyen approprié, notamment par photométrie.

Dans les méthodes immunologiques ci-dessus, utilisant une phase hétérogène, la nature de cette phase solide qui sert de support à la réaction immunologique est très importante car elle détermine la sensibilité de la mesure.

Les supports qui ont été proposés pour le test ELISA sont, notamment, des grains de polyacrylamide activés par le glutaraldéhyde (AVRAMEAS et TERNYNCK, Immunochemistry, 1969, 6, pages 53-65) ; des particules de cellulose activée par le bromure de cyanogène, sur lesquelles des anticorps sont fixés par liaisons covalentes (ENGVALL et PERLMANN, Immunochemistry, 1971, 8, 871-874) ; des tubes de polystyrène sur la surface desquels des antigènes sont fixés par simple adsorption physique (ENGVALL et PERLMANN, J. Immun. 1972, 160, 129-136) ; des disques de papier activé par le bromure de cyanogène et sur lesquels est fixé un anticorps ou un antigène (BRIGHTON et Coll. Scand. 1974, 29, 166-174). Cependant, les supports les plus répandus actuellement sont les surfaces en matière plastique qui se présentent sous forme de microplaques de 96 puits à fond plat, en U ou en V, en polystyrène ou en PVC, ou de barrettes de 8 puits.

Un autre support, formé de billes magnétiques a également été décrit et permet d'envisager l'automatisation des procédés mis en oeuvre.

On peut citer notamment les billes magnétiques décrites dans le Brevet européen 38 730 RHONE POULENC SPECIALITES CHIMIQUES qui décrit des latex de polymères magnétiques, les billes magnétiques décrites dans le Brevet européen 125 995 ADVANCED MAGNETICS INC., les billes magnétiques décrites dans les Brevets français 2 262 805 et 2 454 098 CORNING GLASS WORKS ou les billes décrites dans les Demandes de Brevets européens SERONO DIAGNOSTICS PARTNERS 105 714, 190 006, 238 353, 249 357.

Les différentes billes magnétiques décrites dans les documents précités sont également applicables pour la mise en oeuvre d'un dosage immunologique tel que l'ELISA ou le RIA.

Tant la méthode RIA que la méthode ELISA peuvent être mises en oeuvre de plusieurs manières :

- La méthode indirecte est la plus simple pour doser des anticorps. L'antigène est fixé sur la microplaque (en présence d'albumine pour bloquer les sites non occupés). Le sérum à doser est ajouté et la fixation des anticorps est détectée par l'addition d'anticorps anti-Ig marqués convenablement, notamment couplés à une enzyme et révélés quantitativement au spectrophotomètre après addition du substrat de l'enzyme.

- Une technique de compétition peut être utilisée pour doser les antigènes. Comme dans la technique précédente, c'est l'antigène qui est toujours fixé sur les plaques. Un mélange d'antigène et de faibles quantités d'anticorps est ajouté. La quantité d'anticorps fixée sur la plaque est d'autant plus faible que les molécules d'antigène ont neutralisé plus de molécules d'anticorps.

- Des méthodes en sandwich ou bi-site peuvent être utilisées pour doser des antigènes ou des anticorps. Pour doser des antigènes, les microplaques sont recouvertes de l'anticorps, l'antigène est ajouté puis l'anticorps spécifique, marqué de manière appropriée, notamment couplé à une enzyme, est appliqué et révélé par l'addition du substrat. La technique ne s'applique cependant qu'aux antigènes divalents ou possédant plusieurs déterminants. Pour doser des anticorps, les microplaques sont au contraire recouvertes de l'antigène et on ajoute successivement l'anticorps à doser et l'antigène marqué par une enzyme.

De tels procédés ne sont toutefois pas entièrement automatisables car ils ne permettent pas de mettre en oeuvre un dosage automatique d'au moins une substance dans une pluralité d'échantillons. En effet, la mise en oeuvre de ces différents procédés ne permet d'effectuer que des dosages en série de même type (compétition ou sandwich ou immunocapture) paramètre par paramètre, soit en flux continu, soit en flux discontinu, à cadences faibles.

Or, il est souhaitable, notamment dans les laboratoires d'analyses médicales de fournir des dispositifs automatiques de dosages multi-paramétriques à accès aléatoire, beaucoup plus rapides à réaliser et d'un moindre coût.

Un dosage multi-paramétrique à accès aléatoire permet, notamment sur un échantillon biologique, de réaliser le dosage de vingt paramètres différents et sur l'échantillon suivant de ne réaliser le dosage que de deux paramètres par exemple.

La Demanderesse s'est en conséquence donné pour but de pourvoir à des réactifs de diagnostic immunologique aptes à être mis en oeuvre dans un procédé et un appareil de détermination et/ou de dosage automatique d'au moins une substance, notamment ligands, antiligands, haptènes ou autres molécules biologiques, qui répondent mieux aux nécessités de la pratique que les réactifs de l'Art antérieur, notamment en ce qu'ils permettent d'utiliser beaucoup moins de réactifs, et plus particulièrement des "monoréactifs", d'emploi plus simple, et en ce qu'ils permettent la réalisation de différents types de dosage, tels que compétition, bisite, immunocapture.

La présente invention a pour objet un réactif de diagnostic immunologique constitué de billes magnétiques recouvertes d'une substance se liant spécifiquement à la substance à détecter, en suspension dans un liquide approprié, caractérisé en ce que lesdites billes sont constituées d'une matrice organique renfermant une charge magnétique, lesdites billes ayant un rapport masse de matière magnétisable/masse de matière non magnétisable compris entre 60 et 70 %.

De manière particulièrement avantageuse, la matrice organique est un latex de polymères tel que de polystyrène ou de divinylbenzène.

De préférence, ledit réactif est constitué de billes magnétiques ayant un diamètre inférieur à 1,5 $\mu$m et plus particulièrement compris entre 0,7 et 1,5 $\mu$m.

On peut citer notamment comme billes magnétiques présentant de telles propriétés, les particules décrites dans le Brevet européen 38730, au nom de RHONE-POULENC SPECIALITES CHIMIQUES et dénommées "billes ESTAPOR".

De manière particulièrement avantageuse, ledit réactif est constitué de billes magnétiques recouvertes d'une substance $S_1$ choisie dans le groupe qui comprend les anticorps monoclonaux appropriés, les fragments $F(ab')_2$ d'un anticorps monoclonal approprié, les anticorps polyclonaux appropriés, lesdits anticorps étant dirigés contre la substance à détecter, et les antigènes appropriés, notamment la substance à détecter ou l'un de ses fragments.

Le couplage de ladite substance sur de telles particules magnétiques peut être réalisé selon les techniques de couplage par liaisons covalentes décrites dans l'Art antérieur et notamment selon le mode opératoire décrit par WOOD et coll. dans le Journal of Clinical Chemistry and Clinical Biochemistry, vol. 21, 1983, pp. 789-797.

Selon un autre mode de réalisation dudit réactif, il est constitué d'un prémélange comprenant lesdites billes magnétiques recouvertes d'une substance $S_1$ et un conjugué constitué par un marqueur approprié couplé à une substance $S_2$, identique ou différente de $S_1$ et qui ne réagit pas avec $S_1$, lequel prémélange est réalisé pour la mise en oeuvre du procédé conforme à l'invention.

Selon une disposition avantageuse de ce mode de réalisation, le réactif comprend un conjugué constitué par de la phosphatase alcaline associée à une substance $S_2$ choisie dans le groupe qui comprend les anticorps monoclonaux appropriés, les fragments $F(ab')_2$ d'un anticorps monoclonal approprié, les anticorps polyclonaux appropriés, lesdits anticorps étant dirigés contre la substance à doser et les antigènes appropriés, notamment la substance à doser ou l'un de ses fragments et les complexes antigène-immunoglobuline.

Ledit réactif est notamment constitué d'une solution contenant 0,1 % en rapport P/V de billes magnétiques (soit 1 g en poids sec de billes magnétiques dans 1 litre d'un tampon approprié).

Un tel réactif est particulièrement bien adapté à la mise en oeuvre automatique d'un immunodosage en plusieurs étapes successives d'au moins une substance biologique dans une pluralité d'échantillons biologiques sur un appareil, tels que décrits dans la Demande principale n° 0 454 826, à savoir :

A) un appareil de dosage automatique de type immunologique d'au moins une substance dans une pluralité d'échantillons à analyser, en plusieurs étapes successives constituant un cycle analytique, qui comporte :

(1) un module échantillons constitué par une pluralité de supports de tubes contenant lesdits échantillons, lequel module échantillons est associé à un microprocesseur de commande ;

(2) un module réactionnel constitué par :

. une pluralité de supports de tubes destinés à recevoir successivement une quantité aliquote desdits échantillons et une quantité aliquote d'un réactif approprié et

. un dispositif de lavage comprenant un moyen d'application d'un champ magnétique à la partie inférieure des tubes de réaction et une tête de lavage comportant au moins un moyen d'aspiration du liquide contenu dans lesdits tubes, au moins un moyen de distribution d'un liquide de lavage auxdits tubes et éventuellement au moins un moyen de distribution d'un substrat approprié, lequel module réactionnel est associé à un microprocesseur de commande ;

(3) un module réactifs constitué par une pluralité de supports de tubes contenant le/les réactifs appropriés aux différents dosages à réaliser, au moins l'un desdits réactifs étant sous forme de billes magnétiques ;

(4) un moyen de prélèvement et de distribution des échantillons dans les tubes dudit module réactionnel ;

(5) un moyen de prélèvement et de distribution des réactifs dans les tubes dudit module réactionnel ;

(6) un moyen approprié de lecture de la réaction effectuée dans le module réactionnel et

(7) un système informatique constitué par un ordinateur de commande des différents modules et moyens (1) à (6), et permettant l'exécution d'une succession de cycles analytiques.

Un cycle analytique comprend une série d'étapes constituant au moins une incubation immunologique, et une série d'étapes constituant une incubation de révélation, notamment une incubation enzymatique ; chaque étape correspond à un nombre de positions séquentielles d'un tube dans le module réactionnel, le temps d'arrêt d'un tube dans une position étant constant.

La tête de lavage du dispositif fixe de lavage du module réactionnel, les moyens d'applications d'un champ magnétique, les modules réactions, échantillons et réactifs, et le moyen de décontamination par rinçage des moyens de prélèvement et de distribution des échantillons et des réactifs, sont plus précisément décrits dans la Demande principale n° 0 454 826.

En particulier, le module réactionnel d'un tel appareil comprend un nombre de positions de tubes correspondant à la réalisation d'au moins un cycle analytique comportant la série d'étapes successives suivantes :

- dépôt de l'échantillon à analyser en position 1 ;
- dépôt du réactif approprié en position 4 ;
- préaimantation en position 86 ;
- aimantation et aspiration en position 87 ;
- lavage en position 88 ;
- préaimantation en position 89 ;
- aimantation et aspiration en position 90 ;
- lavage en position 91 ;
- préaimantation en position 92 ;
- aimantation et aspiration en position 93
- lavage en position 94 ;
- préaimantation en position 95 ;
- aimantation et aspiration en position 96 ;
- distribution éventuelle du substrat en position 98 ;
- préaimantation en position 82 ;
- lecture du résultat en position 83.

B) un procédé de dosage immunologique, notamment par compétition ou par une méthode bi-site, en plusieurs étapes successives, d'au moins une substance dans une pluralité d'échantillons à analyser, utilisant comme support solide le réactif selon l'invention (billes magnétiques) qui comporte pour chaque échantillon à analyser :

∗ au moins une incubation immunologique comprenant :

- la distribution successive d'une quantité aliquote d'échantillon puis d'une quantité aliquote de réactif approprié, dans un tube de réaction entraîné en rotation à vitesse constante, au moment où ce dernier est dans la position appropriée auxdites distributions, puis décontamination de chaque système de prélèvement et de distribution,

- la mise en contact de l'échantillon et du réactif approprié pendant un temps convenable correspondant à la durée d'une rotation complète d'un tube de réaction,

- au moins une étape de sédimentation des billes magnétiques sur lesquelles est éventuellement fixée la substance à détecter, par l'application d'un champ magnétique approprié,

- au moins une étape de lavage desdites billes magnétiques, et

∗ une incubation de révélation comprenant :

- éventuellement la distribution et la mise en contact d'un substrat approprié avec lesdites billes, pendant un temps convenable dépendant de la durée de rotation dudit tube de réaction entre ladite distribution et l'étape de lecture du résultat, puis
- la lecture du résultat par tout moyen approprié,

lesdites étapes étant automatisées, constituant un cycle analytique tel que défini ci-dessus et étant déterminées par les rotations synchrones de modules échantillons, réactifs et réactionnel, portant respectivement les tubes contenant les échantillons à analyser, les tubes réactifs et les tubes de réaction destinés à recevoir successivement les quantités aliquotes desdits échantillons et les quantités aliquotes du/des réactifs appropriés comprenant les billes magnétiques.

Les caractéristiques complémentaires concernant ce procédé sont décrites dans la Demande principale n° 0 454 826 (procédé « monoréactif » et procédé « bi-réactif ») :

Pour certaines des substances à doser, un cycle analytique comprenant une seule incubation immunologique et une incubation de révélation est nécessaire ; il s'agit en particulier de l'$\alpha$-féto-protéine (AFP), de la LH, de la FSH, de l'hCG, des IgE et de la prolactine et ce de manière non limitative.

Pour d'autres substances, le cycle analytique comprend deux incubations immunologiques et une incubation de révélation ; il s'agit notamment, et ce de manière non limitative, de la TSH, de la T3, de la T4 totale et de la T4 libre et du cortisol ou bien des anticorps qui apparaissent lors des infections, notamment dues à un parasite (toxoplasmose par exemple), un virus (rubéole, VIH, par exemple) ou à un microorganisme (*Chlamydia* par exemple).

Pour ces dernières substances (anticorps), ce procédé permet de réaliser les dosages en évitant l'étape de pré-dilution habituellement nécessaire.

Le réactif conforme à l'invention, tout en étant constitué d'une phase solide, est manipulable comme un liquide, permettant ainsi un gain de temps et une simplicité, dans l'exécution des différentes opérations nécessaires aux techniques de dosage immunologique et notamment à la méthode ELISA.

Par ailleurs, la charge magnétique élevée des billes qui le constituent autorise, sous l'action d'un champ magnétique approprié, une durée d'aimantation extrêmement courte, permettant la séparation totale desdites billes du surnageant. A titre d'exemple, cette durée d'aimantation est de 15 secondes sous l'action d'un champ magnétique constitué de deux aimants de 1023 tesla environ, autorisant une cadence élevée d'exécution des dosages sur l'analyseur automatique.

De plus, la surface réactionnelle importante des billes magnétiques permet, quelle que soit la substance à doser, que la durée d'une incubation immunologique soit toujours égale à une rotation complète du module réactionnel.

La présente invention a en outre pour objet un kit ou trousse de diagnostic, prêt à l'emploi, caractérisé en ce qu'il est constitué par au moins un réactif de diagnostic immunologique conforme à l'invention.

Outre les dispositions qui précèdent, l'invention comprend encore d'autres dispositions qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

Le réactif conforme à l'invention mis en oeuvre dans un appareil tel que décrit dans la Demande principale n° 454 826 permet notamment de réaliser des dosages immunoenzymatiques comme suit :

**Exemple 1 : Exemple de mise en oeuvre du procédé de dosage immunologique à l'aide d'un appareil conforme à l'invention : dosages dans un même sérum de l'hCG, de la prolactine, des IgG anti-*Chlamydia*, des IgG anti-rubéole, des IgG antitoxoplasmose, dénommé ci-après "plateau grossesse".**

Dans cet exemple, le dosage des IgG et IgM antitoxoplasmose, des IgG antirubéole, des IgG anti-*Chlamydia* nécessite deux incubations immunologiques, la première en présence d'un premier réactif $R_1$ cons-titué de billes magnétiques couplées à une substance $S_1$ se liant spécifiquement à la substance à détecter, la seconde en présence d'un second réactif $R_2$ ou "conjugué" et comprenant un marqueur couplé à une subs-tance $S_2$, identique ou différente de $S_1$ mais qui ne réagit pas avec $S_1$, alors que le dosage de l'hCG et de la prolactine ne nécessite qu'une seule incubation immunologique, avec un prémélange des réactifs $R_1$ et $R_2$ pré-cités.

Les réactifs $R_1$, utilisés dans cet exemple, ont été préparés avec des particules de latex de polystyrène, de diamètre compris entre 0,7 et 1,5 $\mu$l, renfermant une charge magnétique et dont le rapport masse de matière magnétisable/masse de matière non magnétisable est compris entre 60 et 70 %.

Ces billes, dénommées "Billes ESTAPOR" sont fournies par RHONE-POULENC SPECIALITES CHIMIQUES.

Le couplage de la substance $S_1$ (anticorps monoclonaux, fragments F(ab')$_2$ d'anticorps monoclonaux, an-ticorps polyclonaux, ...) a été réalisé selon le mode opératoire décrit par WOOD et coll. dans Journal of Clinical Chemistry and Clinical Biochemistry, vol. 21, 1983, pp. 789-797.

Les réactifs $R_2$ ou conjugués ont été préparés selon un mode opératoire analogue à celui décrit par AVRAMEAS dans Immunochemistry, 5, 43, 1969.

Le marqueur choisi est la phosphatase alcaline (PAL). Le substrat de révélation est le para-nitro-phényl phosphate (PNPP).

Le Tableau I suivant présente la composition et la concentration des réactifs utilisés.

TABLEAU I

| Substances à doser | Réactif $R_1$ | | Réactif $R_2$ | |
|---|---|---|---|---|
| hCG | Billes ESTAPOR-fragment F(ab')$_2$ d'un anticorps monoclonal anti-hCG | *Solution à 0,1 % P/V | PAL-fragment F(ab')$_2$ d'un anticorps monoclonal anti-hCG | **Solution à 5 mg/l |
| Prolactine | Billes ESTAPOR-fragment F(ab')$_2$ d'un anticorps monoclonal anti-prolactine | *Solution à 0,1 % P/V | PAL-fragment F(ab')$_2$ d'un anticorps monoclonal anti-prolactine | **Solution à 5 mg/l |
| Toxo G | Billes ESTAPOR-antigène toxoplasmique | *Solution à 0,1 % P/V | PAL-anticorps polyclonal anti IgG humaine | **Solution à 50 µg/l |
| Rubéole | Billes ESTAPOR-antigène toxoplasmique | *Solution à 0,1 % P/V | PAL-anticorps polyclonal anti IgG humaine | **Solution à 50 µg/l |
| Chlamydia | Billes ESTAPOR-antigène chlamydia | *Solution à 0,1 % P/V | PAL-anticorps polyclonal anti IgG humaine | **Solution à 50 µg/l |
| Toxo M | Billes ESTAPOR-fragment F(ab')$_2$ d'anticorps monoclonal anti-toxoplasmose | *Solution à 0,1 % P/V | Antigène toxoplasmique purifié marqué par une Ig animale-PAL | **Solution à 20mg/l |

\* solution contenant 1g en poids sec de billes par litre de tampon PBS 0,1 M - pH 7,3 (BSA 5g/l-NaN$_3$ 1g/l).

\*\* tampon Tris 0,1 M - pH 8 (BSA 5 g/l-Mg Cl$_2$ 1g/l NaN$_3$ 1g/l).

Le Tableau II suivant présente les quantités nécessaires exprimées en µl des différents produits à distribuer, en fonction de la substance à doser :

TABLEAU II

| Substance | Sérum | Flush | Réactif 1 | Réactif 2 | PNPP |
|-----------|-------|-------|-----------|-----------|------|
| hCG | 100 | 50 | 300 | | 500 |
| Prolactine | 25 | 50 | 300 | | 500 |
| Toxo G | 10 | 50 | 300 | 400 | 500 |
| Rubéole | 10 | 50 | 300 | 300 | 500 |
| Chlamydia | 10 | 50 | 300 | 400 | 500 |
| Toxo M | 10 | 50 | 300 | 400 | 500 |

Dans cet exemple, le module réactifs E comporte :
- en position 1, le monoréactif hCG comprenant des billes "ESTAPOR" recouvertes d'un fragment $F(ab')_2$ d'un anticorps monoclonal dirigé contre l'hCG et un conjugué phosphatase alcaline (PAL)-fragment $F(ab')_2$ d'un anticorps monoclonal dirigé contre l'hCG, différent du premier ;
- en position 2, le monoréactif prolactine comprenant des billes "ESTAPOR" recouvertes d'un fragment $F(ab')_2$ d'un anticorps monoclonal dirigé contre la prolactine et un conjugué phosphatase alcaline (PAL)-fragment $F(ab')_2$ d'un anticorps monoclonal dirigé contre la prolactine, différent du premier ;
- en position 3, le réactif $R_1$ pour le dosage des IgG antitoxoplasmose comprenant des billes "ESTAPOR" recouvertes d'un antigène toxoplasmique ;
- en position 4, le réactif $R_4$ pour le dosage des IgG antitoxoplasmose, constitué d'un conjugué phosphatase alcaline (PAL)-anticorps polyclonal anti-IgG humaine
- en position 5, le réactif $R_1$ contre la rubéole comprenant des billes magnétiques recouvertes d'un antigène rubéolique ;
- en position 6, le réactif $R_2$ contre la rubéole, constitué d'un conjugué phosphatase alcaline (PAL)-anticorps polyclonal anti-IgG humaine ;
- en position 7, le réactif $R_1$ anti-*Chlamydia* comprenant des billes magnétiques recouvertes d'un antigène chlamydial ;
- en position 8, le réactif $R_2$ anti-*Chlamydia,* constitué d'un conjugué phosphatase alcaline (PAL)-anticorps polyclonal anti-IgG humaine ;
- en position 9, le réactif $R_1$ pour le dosage des IgM antitoxoplasmose, comprenant des billes magnétiques recouvertes d'un fragment $F(ab')_2$ d'un anticorps monoclonal anti-toxoplasmose ;
- en position 10, le réactif $R_2$ pour le dosage des IgM antitoxoplasmose, constitué d'un complexe formé d'un antigène toxoplasmique purifié, marqué par une Ig d'origine animale, conjuguée à la phosphatase alcaline
- et le module échantillons A comprend le tube de sérum à analyser.

Six tubes de réaction vont donc recevoir, tour à tour, en position 1 du module réactionnel C, une quantité appropriée de sérum et une quantité appropriée de "flush" distribuées par le bras B de prélèvement et de distribution des échantillons. Puis, lorsque chacun des tubes arrive en position 4 sur le module réactionnel C, il reçoit une quantité appropriée :
- pour le tube 1, de monoréactif hCG,
- pour le tube 2, de monoréactif prolactine,
- pour le tube 3, de réactif $R_1$ IgG anti-toxoplasmose,
- pour le tube 4, de réactif $R_1$ IgG anti-rubéole,
- pour le tube 5, de réactif $R_1$ IgG anti-chlamydia,
- pour le tube 6, de réactif $R_1$ IgM anti-toxoplasmose.

Pour les dosages de l'hCG et de la prolactine ne nécessitant qu'une seule incubation immunologique, la séquence analytique se poursuit jusqu'à distribution du substrat lorsque les tubes arrivent en position 98 du module réactionnel, ce qui correspond à l'exécution d'un tour complet de rotation dudit module. Au cours du tour suivant de rotation, se réalisent une préaimantation en position 82 et la lecture de chaque réaction en position 83, à trois longueurs d'onde différentes (405, 450 et 604 nm).

Pour les dosages nécessitant deux incubations immunologiques, la première incubation est réalisée de la même manière que ci-dessus, excepté qu'il n'y a pas de distribution de substrat en position 98 ; en effet, lorsque les tubes arrivent en position 4 du second tour de rotation du module réactionnel C, ils reçoivent respectivement une quantité appropriée :
- pour le tube 3, de réactif $R_2$ IgG anti-toxoplasmose,
- pour le tube 4, de réactif $R_2$ IgG anti-rubéole,
- pour le tube 5, de réactif $R_2$ IgG anti-chlamydia,
- pour le tube 6, de réactif $R_2$ IgM anti-toxoplasmose,

pour la réalisation d'une seconde incubation immunologique, qui se poursuit jusqu'à distribution du substrat en position 98 du second tour de rotation du module, puis une préaimantation et la lecture du résultat s'effectuent respectivement en position 82 et 83 du troisième tour de rotation dudit module.

Le Tableau III suivant présente le cycle analytique de chacune des substances :

TABLEAU III

| POSITIONS DU MODULE REACTIONNEL | | hCG (TUBE 1) PROLACTINE (TUBE 2) | TOXO G (TUBE 3) TOXO M (TUBE 4) CHLAMYDIA (TUBE 5) RUBEOLE (TUBE 6) |
|---|---|---|---|
| | 1 | Dépôt échantillon + flush | Dépôt échantillon + flush |
| | 4 | Dépôt Monoréactif ($R_1$ + $R_2$) | Dépôt réactif $R_1$ |
| | 86 | Préaimantation | Préaimantation |
| | 87 | Aimantation | Aimantation |
| | 88 | Lavage | Lavage |
| | 89 | Préaimantation | Préaimantation |
| 1er tour de | 90 | Aimantation | Aimantation |
| rotation du | 91 | Lavage | Lavage |
| module réac- | 92 | Préaimantation | Préaimantation |
| tionnel | 93 | Aimantation | Aimantation |
| | 94 | Lavage | Lavage |
| | 95 | Préaimantation | Préaimantation |
| | 96 | Aimantation | Aimantation |
| | 98 | Dépôt substrat | |
| | 4 | | Dépôt réactif $R_2$ |
| | 82 | Préaimantation | |
| | 83 | Lecture résultat | |
| | 86 | | Préaimantation |
| | 87 | | Aimantation |
| | 88 | | Lavage |
| | 89 | | Préaimantation |
| 2ème tour de | 90 | | Aimantation |
| rotation du | 91 | | Lavage |
| module réac- | 92 | | Préaimantation |
| tionnel | 93 | | Aimantation |
| | 94 | | Lavage |
| | 95 | | Préaimantation |
| | 96 | | Aimantation |
| | 98 | | Dépôt substrat |
| 3ème tour de | 82 | | Préaimantation |
| rotation du | 83 | | Lecture résultat |
| module réactionnel | | | |
| TEMPS DE SORTIE DU 1er RESULTAT | | 30 MINUTES | 47 MINUTES |

Pour un temps d'arrêt des tubes de réaction dans chaque position du module réactionnel C de 10 secondes, le premier résultat est obtenu au bout de 30 minutes pour les dosages ne nécessitant qu'une seule incubation immunologique et au bout de 47 minutes pour les dosages nécessitant deux incubations immunologiques.

Ainsi, le temps nécessaire à la réalisation de 100 dosages nécessitant une incubation immunologique est d'environ 50 minutes et le temps nécessaire à la réalisation de 100 dosages nécessitant deux incubations immunologiques est d'environ 65 minutes.

L'exemple qui vient d'être décrit porte sur le dosage de plusieurs substances différentes présentes dans le même échantillon de sérum. On comprendra qu'il est possible de ne doser, dans un autre échantillon de sérum, qu'une seule substance, telle que la prolactine par exemple. Il s'agit donc bien d'un dosage multiparamétrique à accès aléatoire, permettant de détecter un nombre différent de substances dans chaque échantillon.

Il faut noter que le procédé décrit ci-dessus, mis en oeuvre dans l'appareil conforme à l'invention, a l'avantage de comporter des étapes d'incubation de même durée, quelle que soit la substance recherchée, la concentration de conjugué approprié à chaque substance étant ajustée de manière que les étapes d'incubation soient effectivement de même durée.

### Exemple 2 : dosage de 15 paramètres différents.

Le tableau IV ci-après montre les quantités des différentes substances à ajouter :

|  | *SERUM | *FLUSH | *Réactif 1 | *Réactif 2 | *SUBSTRAT |
|---|---|---|---|---|---|
| AFP | 20 | 50 | 3 0|0 |  | 500 |
| LH | 100 | 50 | 3 0|0 |  | 500 |
| FSH | 100 | 50 | 3 0|0 |  | 500 |
| hCG | 100 | 50 | 3 0|0 |  | 500 |
| IgE | 20 | 50 | 3 0|0 |  | 500 |
| PRL | 25 | 50 | 3 0|0 |  | 500 |
| CHLAMYDIA | 10 | 50 | 300 | 400 | 500 |
| CORTISOL | 100 | 50 | 100 | 150 | 500 |
| RUBEOLE | 10 | 50 | 300 | 300 | 500 |
| T3 | 150 | 50 | 200 | 400 | 500 |
| T4 | 100 | 50 | 200 | 400 | 500 |
| T4 libre | 100 | 50 | 150 | 200 | 500 |
| TOXO G | 10 | 50 | 300 | 400 | 500 |
| TOXO M | 10 | 50 | 300 | 400 | 500 |
| TSH | 150 | 50 | 150 | 400 | 500 |

*tous les volumes sont exprimés en µl.

De manière générale, le réactif utilisé pour le dosage des substances qui ne nécessitent qu'une incubation immunologique est constitué par des billes recouvertes d'un fragment F(ab')$_2$ d'un anticorps monoclonal dirigé contre la substance à doser et d'un conjugué PAL-F(ab')$_2$ d'un anticorps monoclonal différent du premier, dirigé contre la substance à doser.

Pour ce qui concerne les substances qui nécessitent deux incubations immunologiques :
- cortisol : le premier réactif est constitué de billes recouvertes d'anticorps polyclonaux anti-cortisol et le deuxième réactif est constitué de cortisol marqué à la PAL
- T3 totale, T3 libre, T4 totale et T4 libre : le premier réactif est constitué de billes recouvertes d'anticorps monoclonaux anti-T3 ou T4 sous forme F(ab')$_2$ et le deuxième réactif est constitué de T3 ou T4 marquée à la PAL ;
- TSH : le premier réactif est constitué de billes recouvertes d'anticorps monoclonaux anti-TSH sous forme F(ab')$_2$ et le deuxième réactif est constitué de fragment F(ab')$_2$ d'un autre anticorps monoclonal dirigé contre la TSH marquée à la PAL.

Comme dans l'exemple 1, on peut programmer le dosage d'un nombre de paramètres différents pour chaque échantillon à analyser.

**Exemple 3 : Rôle de la masse magnétisable des réactifs, dans la mise en oeuvre du procédé.**

Si l'on procède à un dosage tel que décrit à l'exemple 1 ci-dessus avec des billes magnétiques (DYNAL S.A.) ayant les caractéristiques suivantes :

| | |
|---|---|
| Diamètre : | $2,8 \ \mu m \pm 0,2 \ \mu m$ |
| Coefficient de variation | 5 % (habituellement < 2 %) |
| Masse de matière magnétisable | $12 \pm 2$ %, |

ces dernières, dont la masse de matière magnétisalbe est de 12 %, sont aspirées lors du lavage, et en conséquence aucun résultat ne peut être obtenu au contraire, les billes magnétiques conformes à l'invention, dont la masse de matière magnétisable est supérieure à 45 %, permettent d'obtenir des résultats dans des délais très courts.

Ainsi que cela ressort de ce qui précède, l'invention ne se limite nullement à ceux de ses modes de mise en oeuvre, de réalisation et d'application qui viennent d'être décrits de façon plus explicite ; elle en embrasse, au contraire, toutes les variantes qui peuvent venir à l'esprit du technicien en la matière, sans s'écarter du cadre ni de la portée de la présente invention.

**Revendications**

1°) Réactif de diagnostic immunologique constitué de billes magnétiques recouvertes d'une substance se liant spécifiquement à la substance à détecter, en suspension dans un liquide approprié, caractérisé en ce que lesdites billes sont constituées d'une matrice organique renfermant une charge magnétique, lesdites billes ayant un rapport masse de matière magnétisable/masse de matière non magnétisable compris entre 60 et 70 %.

2°) Réactif selon la revendication 1, caractérisé en ce que la matrice organique est un latex de polymères tel que de polystyrène ou de divinylbenzène.

3°) Réactif selon la revendication 1 ou la revendication 2, caractérisé en ce que ledit réactif est constitué de billes magnétiques ayant un diamètre inférieur à $1,5 \ \mu m$ et plus particulièrement compris entre 0,7 et 1,5 $\mu m$.

4°) Réactif selon l'une quelconque des revendications 1 à 3, caractérisé en ce que ledit réactif est constitué de billes magnétiques recouvertes d'une substance $S_1$ choisie dans le groupe qui comprend les anticorps monoclonaux appropriés, les fragments $F(ab')_2$ d'un anticorps monoclonal approprié, les anticorps polyclonaux appropriés, lesdits anticorps étant dirigés contre la substance à détecter, et les antigènes appropriés, notamment la substance à détecter ou l'un de ses fragments.

5°) Réactif selon l'une quelconque des revendications 1 à 4, caractérisé en ce qu'il est constitué d'un prémélange comprenant lesdites billes magnétiques recouvertes d'une substance $S_1$ et un conjugué constitué par un marqueur approprié couplé à une substance $S_2$, identique ou différente de $S_1$ et qui ne réagit pas avec $S_1$, lequel prémélange est réalisé pour la mise en oeuvre du procédé selon l'une quelconque des revendications 12 à 20.

6°) Réactif selon la revendication 5, caractérisé en ce qu'il comprend un conjugué constitué par de la phosphatase alcaline associée à une substance $S_2$ choisie dans le groupe qui comprend les anticorps monoclonaux appropriés, les fragments $F(ab')_2$ d'un anticorps monoclonal approprié, les anticorps polyclonaux appropriés, lesdits anticorps étant dirigés contre la substance à doser et les antigènes appropriés, notamment la substance à doser ou l'un de ses fragments et les complexes antigène-immunoglobuline.

7°) Kit ou trousse de diagnostic, prêt à l'emploi, caractérisé en ce qu'il est constitué par au moins un réactif de diagnostic immunologique selon l'une quelconque des revendications 1 à 6.